# FASCICULE DE BREVET EUROPEEN

(11) **EP 2 364 173 B1**
(45) Date de publication et mention de la délivrance du brevet: **06.11.2013**
(21) Numéro de dépôt: 09752428.4
(22) Date de dépôt: 29.09.2009
(51) Int. Cl.: A61M 15/00

(54) **DISPOSITIF D'INHALATION DE POUDRE**
PULVERINHALATIONSVORRICHTUNG
POWDER INHALATION DEVICE

(30) Priorité: 30.09.2008 FR 0856561
(43) Date de publication de la demande: 14.09.2011
(73) Titulaire: Aptar France SAS, 27110 Le Neubourg (FR)
(72) Inventeur: BAILLET, Matthieu, F-76240 Bonsecours (FR)
(74) Mandataire: CAPRI
(86) Numéro de dépôt international: PCT/FR2009/051848
(87) Numéro de publication internationale: WO 2010/037965

(56) Documents cités:
- EP-A- 1 905 470
- WO-A-01/26720
- WO-A-2007/118490
- FR-A- 2 881 117
- FR-A- 2 909 641
- US-A- 5 469 843

## Description

La présente invention concerne un dispositif d'inhalation de poudre, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation.

Pour assurer une distribution finement pulvérisée de la poudre, le document US 6 715 486 décrit une chambre de dispersion contenant une ou plusieurs billes entraînées en rotation par l'écoulement d'air et de poudre se dirigeant du réservoir ouvert vers l'orifice de distribution. Cette chambre de dispersion assure une bonne désagglomération de la poudre, et a un effet positif sur la résistance à l'écoulement en la diminuant. Les effets de cette chambre à bille sont toutefois relativement sensibles à l'orientation de l'inhalateur au moment de l'inhalation, avec des propriétés de rendement, de variabilité ou de résistance qui pourront être affectés en cas d'orientation non optimale, correspondant à l'inhalateur maintenu autrement que verticalement. De plus, l'utilisation de billes complique l'assemblage et le rend plus coûteux.

Les documents WO 01/26720, FR-2 881 117, FR-2 909 641, WO 2007/118490 et US-5 469 843 décrivent des dispositifs de l'art antérieur.

La présente invention a pour but de fournir un dispositif d'inhalation de poudre, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel inhalateur qui assure une bonne précision de dosage et une bonne reproductibilité de dosage à chaque actionnement, indépendamment de l'orientation de l'inhalateur.

La présente invention a donc pour objet un dispositif d'inhalation de poudre, comportant un corps pourvu d'un orifice de distribution, une pluralité de réservoirs prédosés contenant chacun une dose de poudre à distribuer, et des moyens d'ouverture de réservoir pour ouvrir un réservoir à chaque actionnement, lesdits moyens d'ouverture étant des moyens de perçage comportant une aiguille adaptée à percer un réservoir prédosé à chaque actionnement le dispositif comportant un canal de dispersion comportant une entrée reliée auxdits moyens de perçage et recevant la dose de poudre à partir dudit réservoir ouvert et une sortie reliée audit orifice de distribution, lesdits moyens de perçage étant commandés par l'inhalation de l'utilisateur, de sorte qu'un réservoir prédosé est simultanément percé et vidé, la poudre entraînée par l'écoulement d'inhalation traversant ledit canal de dispersion avant d'être expulsée à travers l'orifice de distribution, ledit canal de dispersion comportant deux parties de canal coudées, une première partie de canal coudée reliée à ladite entrée par une première partie de canal sensiblement rectiligne, et une seconde partie de canal coudée reliée à ladite sortie par une seconde partie de canal sensiblement rectiligne, lesdites deux parties de canal coudées étant reliées l'une à l'autre par une troisième partie de canal sensiblement rectiligne, la section transversale de ladite troisième partie de canal sensiblement rectiligne étant inférieure à la section transversale de ladite première partie de canal sensiblement rectiligne.

Avantageusement, lesdites première et seconde parties de canal sensiblement rectilignes sont sensiblement parallèles.

Avantageusement, ladite première partie de canal coudée forme un angle supérieur ou égal à 90°, de préférence d'environ 100°.

Avantageusement, ladite seconde partie de canal coudée forme un angle supérieur ou égal à 90°, de préférence d'environ 100°.

Avantageusement, ledit canal de dispersion comporte une partie de fond et une partie de couvercle.

Avantageusement, l'entrée et la première partie de canal sensiblement rectiligne du canal de dispersion sont formées sur la partie de fond, la sortie et la seconde partie de canal sensiblement rectiligne sont formées sur la partie de couvercle, et lesdites deux parties de canal coudées et ladite troisième partie de canal sensiblement rectiligne sont formées à la fois sur la partie de fond et sur la partie de couvercle.

Avantageusement, ladite première partie de canal sensiblement rectiligne, et/ou ladite seconde partie de canal sensiblement rectiligne et/ou ladite troisième partie de canal sensiblement rectiligne ont une section transversale sensiblement constante sur leur longueur respective.

Avantageusement, ladite seconde partie de canal sensiblement rectiligne comporte des moyens de restriction.

Ces caractéristiques et avantages et d'autres de la présente invention apparaîtront plus clairement au cours de la description détaillée suivante, 5 faite en référence aux dessins joints, donnés à titre d'exemples non limitatifs, sur lesquels
- la figure 1 est une vue schématique en section transversale d'un inhalateur de poudre,
- la figure 2 représente une vue schématique en perspective éclatée d'une partie du dispositif d'inhalation, selon une variante de réalisation qui n'est pas couverte par l'invention,
- la figure 3 est une vue similaire à celle de la figure 2, montrant un mode de réalisation avantageux de l'invention, et
- la figure 4 représente une vue schématique en section transversale de côté de la partie du dispositif d'inhalation de la figure 3.

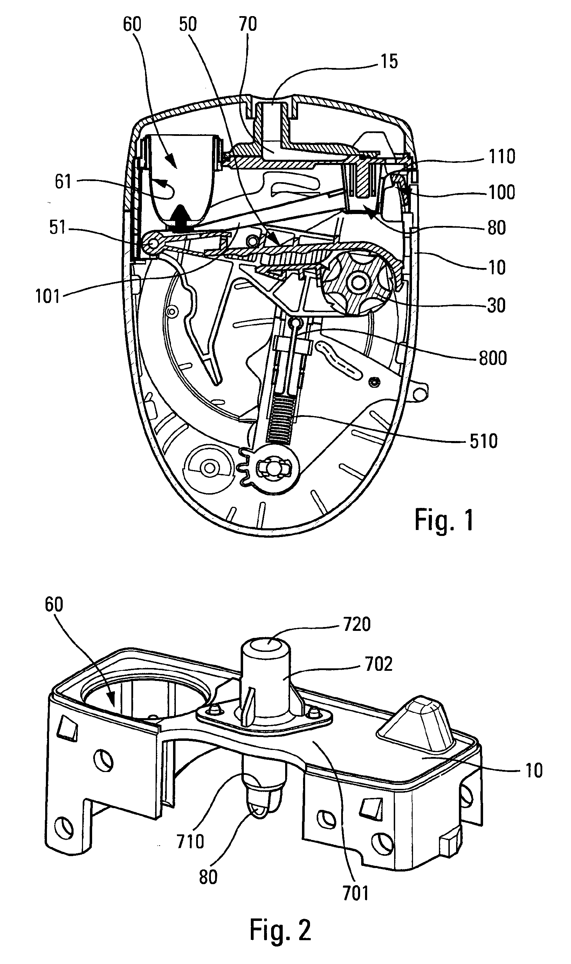

Sur la figure 1 est représentée une variante de réalisation avantageuse d'un inhalateur de poudre sèche. Cet inhalateur comporte un corps 10 sur lequel peuvent être montées coulissantes ou pivotantes deux parties formant un capot (non représentées) adaptées à être ouvertes pour ouvrir et charger le dispositif. Le corps 10 peut être de forme environ arrondie, mais il pourrait avoir tout autre forme appropriée. Le corps 10 comporte un embout buccal ou d'inhalation définissant un orifice de distribution 15 à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable. En variante, le dispositif pourrait comporter un seul capot au lieu de deux.

A l'intérieur du corps 10, il est prévu une bande (non représentée) de réservoirs individuels prédosés, également appelés blisters, réalisée sous forme d'une bande souple allongée sur laquelle les blisters sont disposés les uns derrière les autres, de manière connue. Avant la première utilisation, la bande de blister peut être enroulée à l'intérieur du corps 10, de préférence dans une partie de stockage, et des premiers moyens de déplacement de bande 30 sont prévus pour progressivement dérouler et faire avancer cette bande de blister. Des seconds moyens de déplacement 50, 51 sont prévus pour amener un réservoir individuel ou blister respectif dans une position de distribution à chaque actionnement du dispositif. La partie de bande comportant les réservoirs vides est avantageusement adaptée à s'enrouler dans un autre endroit dudit corps 10, de préférence une partie de réception.

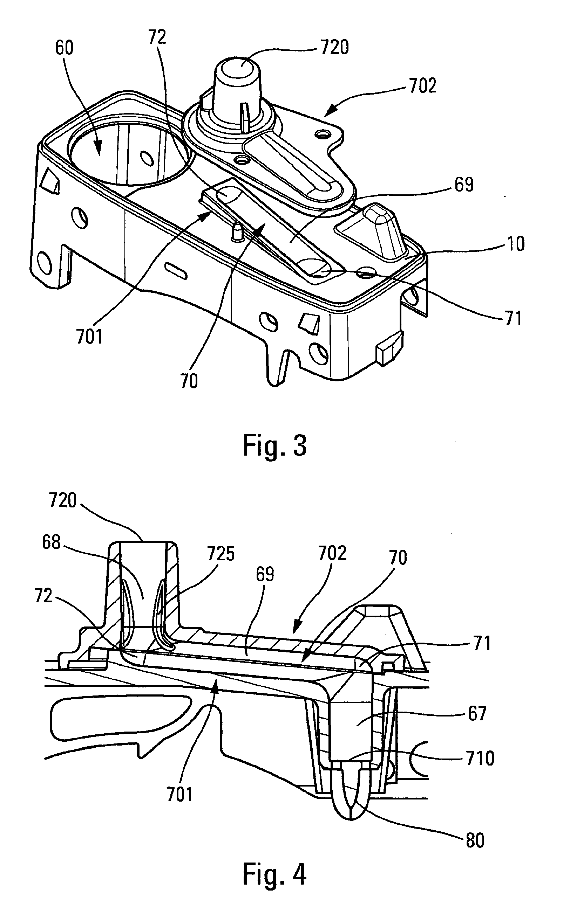

L'inhalateur comporte des moyens d'ouverture de réservoir 80 (qui ne sont indiqués sur la figure 1 que de manière très schématique, et qui sont mieux visibles sur les figures 2 et 4) comportant des moyens de perçage de la couche de fermeture des blisters. Ces moyens de perçage comportent une aiguille, de préférence fixe par rapport au corps 10, et contre laquelle un une aiguille, de préférence fixe par rapport au corps 10, et contre laquelle un blister respectif est déplacé à chaque actionnement par les seconds moyens de déplacement. Le blister est alors percé par ladite aiguille, qui pénètre dans ledit blister pour expulser la poudre au moyen du flux d'inhalation de l'utilisateur.

Les premiers moyens de déplacement sont adaptés à faire avancer la bande de blisters avant et/ou pendant et/ou après chaque actionnement du dispositif. Les seconds moyens de déplacement sont adaptés à déplacer le réservoir à vider contre lesdits moyens de perçage lors de l'actionnement. Ces seconds moyens de déplacement peuvent être sollicités, via des moyens de chargement 800, par un élément élastique 510, tel qu'un ressort ou tout autre élément élastique équivalent, ledit élément élastique pouvant être préchargé lors de l'ouverture du dispositif. De préférence, les premiers moyens de déplacement comportent une roue d'indexage 30 qui reçoit et guide les blisters. Une rotation de cette roue d'indexage fait avancer la bande de blister. Dans une position angulaire particulière, un réservoir donné est toujours en position face aux moyens d'ouverture. Les seconds moyens de déplacement peuvent comporter un élément de support 50 rotatif autour d'un axe de rotation 51, ladite roue d'indexage 30 étant montée rotative sur ledit élément de support.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage ne peut pas se déplacer vers l'aiguille car les seconds moyens de déplacement sont retenus par des moyens de blocage 100, 110 appropriés. De préférence, c'est lors de l'inhalation par l'utilisateur à travers l'embout buccal que ces moyens de blocage sont débloqués, ce qui provoque alors le pivotement dudit élément de support 50 et donc le déplacement de ladite roue d'indexage 30 en direction de l'aiguille,et donc l'ouverture d'un réservoir.

L'orientation optimale de l'inhalateur lors de son utilisation correspond à une position sensiblement verticale avec l'orifice distribution 15 dirigé vers le haut, comme représenté sur la figure 1. Néanmoins, l'inhalateur selon la présente invention peut être utilisé dans n'importe quelle position.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation, qui comporte avantageusement une unité 60 déplaçable et/ou déformable sous l'effet de l'inhalation, cette unité étant adaptée à libérer les moyens de blocage 100, 110, par exemple via une tige 101. Cette unité comprend avantageusement une chambre d'air déformable 61. L'inhalation de l'utilisateur provoque la déformation de ladite chambre d'air déformable, permettant ainsi de libérer lesdits moyens de blocage et donc de permettre le déplacement des seconds moyens de déplacement, et donc d'un réservoir respectif vers sa position d'ouverture. Le réservoir n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

Après l'inhalation, lorsque l'utilisateur referme le dispositif, tous les composants reviennent vers leur position de repos initiale. Le dispositif est alors prêt pour un nouveau cycle d'utilisation.

Selon l'invention, l'inhalateur comporte un canal de dispersion 70 qui est destinée à recevoir la dose de poudre après ouverture d'un réservoir respectif. Ce canal de dispersion 70 comporte une entrée 710, directement reliée aux moyens d'ouverture de réservoir, et une sortie 720, directement reliée à l'orifice de distribution 15. Ainsi, aucune chambre de dispersion, avec ou sans billes, n'est prévue entre l'entrée 710 et la sortie 720 dudit canal de dispersion 70, et donc entre les moyens de perçage 80 et l'orifice de distribution 15. Cette mise en oeuvre simplifie donc la fabrication et l'assemblage du dispositif.

L'exemple de la figure 2, qui n'est pas couvert par l'invention, montre un canal sensiblement rectiligne entre l'entrée 710 et la sortie 720 du canal. Avantageusement, la section transversale de ce canal de dispersion 70 est sensiblement constante sur toute la longueur dudit canal.

Le canal de dispersion 70 comporte au moins une première partie de canal coudée 71 reliée à ladite entrée 710 par une première partie de canal sensiblement rectiligne 67, de préférence de section transversale constante sur sa longueur. Le canal de dispersion 70 comporte avantageusement également une seconde partie de canal coudée 72 reliée à ladite sortie 720 par une seconde partie de canal sensiblement rectiligne 68, qui peut aussi être de section transversale constante. Les figures 1, 3 et 4 montrent des exemples de ces deux parties de canal coudées. Ces deux parties de canal coudées 71 et 72 peuvent être reliées l'une à l'autre par une troisième partie de canal sensiblement rectiligne 69, qui est également de préférence de section transversale constante. Selon l'invention, cette troisième partie de canal sensiblement rectiligne 69 comporte une section transversale inférieure à celle de la première partie de canal sensiblement rectiligne 67, avec pour effet une accélération de l'écoulement d'air et de poudre la traversant. En variante, comme représenté très schématiquement sur la figure 1, la troisième partie de canal sensiblement rectiligne 69 pourrait aussi avoir une section transversale croissante en direction de la seconde partie de canal coudée 72. La seconde partie de canal sensiblement rectiligne 68 peut avoir une section transversale similaire à celle de la première partie de canal sensiblement rectiligne 67, comme visible sur la figure 4. Elle peut aussi comporter des moyens de restrictions 725, par exemple une ou plusieurs projections saillante vers l'intérieur, pour perturber l'écoulement d'air et poudre et favoriser la désagglomération de ladite poudre. Les dimensions et sections des diverses parties de canal peuvent être optimisées en fonction de l'agencement des différentes parties constitutives de l'inhalateur, et en fonction des performances souhaitées.

Avantageusement, l'angle formé par les deux parties coudées 71 et 72 est supérieur ou égal à 90°, de préférence d'environ 100°. Comme visible sur les figures 3 et 4, les première et seconde parties de canal sensiblement rectilignes 67 et 68 peuvent être sensiblement parallèles, et la troisième partie de canal sensiblement rectiligne 69 peut être légèrement inclinée entre les deux parties de canal coudées 71 et 72. Ceci procure des excellents rendements, avec plus de 95 % de la poudre contenue initialement dans un réservoir qui est transmise à la sortie 720 dudit canal de dispersion 70. De même, on obtient une très bonne reproductibilité de dosage lors d'actionnements successifs. Les dimensions et orientations des différentes parties de canal peuvent bien entendu être optimisées selon les spécificités de l'inhalateur, pour maximiser les performances de celui-ci.

Avantageusement, le canal de dispersion 70 comporte une partie de fond 701 et une partie de couvercle 702. De préférence, comme visible sur les figures 3 et 4, l'entrée 710 et la première partie de canal rectiligne 67 sont formées exclusivement sur la partie de fond 701. De même, la sortie 720 et la seconde partie de canal sensiblement rectiligne 68 sont de préférence formées exclusivement sur la partie de couvercle 702. Les deux parties de canal coudées 71 et 72 et la troisième partie de canal sensiblement rectiligne 69 sont de préférence formées sur les deux parties de fond et de couvercle. Ceci simplifie le moulage et l'assemblage.

La présente invention permet donc de fournir un inhalateur de poudre sèche qui procure notamment les fonctions suivantes :
■ une pluralité de doses individuelles de poudre stockées dans des réservoirs individuels étanches, par exemple 30 ou 60 doses stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
■ Une dispersion efficace de la poudre avant son expulsion, pour limiter les rétentions de poudre et pour garantir une bonne précision et reproductibilité de dosage à chaque actionnement, même en cas d'orientation non optimale de l'inhalation.

D'autres fonctions sont également fournies par le dispositif de l'invention tel que cela a été décrit précédemment. Il est à noter que les différentes fonctions, même si elles ont été représentées comme prévues simultanément sur les modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, indépendamment de la forme du canal de dispersion, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées.

## Revendications

1. Dispositif d'inhalation de poudre, comportant un corps (10) pourvu d'un orifice de distribution (15), une pluralité de réservoirs prédosés contenant chacun une dose de poudre à distribuer, et des moyens d'ouverture de réservoir (80) pour ouvrir un réservoir à chaque actionnement, lesdits moyens d'ouverture étant des moyens de perçage comportant une aiguille adaptée à percer un réservoir prédosé à chaque actionnement, le dispositif comportant un canal de dispersion (70) comportant une entrée (710) reliée auxdits moyens de perçage (80) et recevant la dose de poudre à partir dudit réservoir ouvert et une sortie (720) reliée audit orifice de distribution (15), lesdits moyens de perçage étant commandés par l'inhalation de l'utilisateur, de sorte qu'un réservoir prédosé est simultanément percé et vidé, la poudre entraînée par l'écoulement d'inhalation traversant ledit canal de dispersion (70) avant d'être expulsée à travers l'orifice de distribution (15), **caractérisé en ce que** ledit canal de dispersion (70) comporte deux parties de canal coudées (71, 72), une première partie de canal coudée (71) reliée à ladite entrée (710) par une première partie de canal sensiblement rectiligne (67), et une seconde partie de canal coudée (72) reliée à ladite sortie (720) par une seconde partie de canal sensiblement rectiligne (68), lesdites deux parties de canal coudées (71, 72) étant reliées l'une à l'autre par une troisième partie de canal sensiblement rectiligne (69), la section transversale de ladite troisième partie de canal sensiblement rectiligne (69) étant inférieure à la section transversale de ladite première partie de canal sensiblement rectiligne (67).

2. Dispositif selon la revendication 1, dans lequel lesdites première et seconde parties de canal sensiblement rectilignes (67, 68) sont sensiblement parallèles.

3. Dispositif selon les revendications 1 ou 2, dans lequel ladite première partie de canal coudée (71) forme un angle supérieur ou égal à 90°, de préférence d'environ 100°.

4. Dispositif selon l'une quelconque des revendications précédentes, dans lequel ladite seconde partie de canal coudées (72) forme un angle supérieur ou égal à 90°, de préférence d'environ 100°.

5. Dispositif selon l'une quelconque des revendications précédentes, dans lequel ledit canal de dispersion (70) comporte une partie de fond (701) et une partie de couvercle (702).

6. Dispositif selon la revendication 5, dans lequel l'entrée (710) et la première partie de canal sensiblement rectiligne (67) du canal de dispersion (70) sont formées sur la partie de fond (701), la sortie (720) et la seconde partie de canal sensiblement rectiligne (68) sont formées sur la partie de couvercle (702), et lesdites deux parties de canal coudées (71, 72) et ladite troisième partie de canal sensiblement rectiligne (69) sont formées à la fois sur la partie de fond (701) et sur la partie de couvercle (702).

7. Dispositif selon l'une quelconque des revendications précédentes, dans lequel ladite première partie de canal sensiblement rectiligne (67), et/ou ladite seconde partie de canal sensiblement rectiligne (68) et/ou ladite troisième partie de canal sensiblement rectiligne (69) ont une section transversale sensiblement constante sur leur longueur respective.

8. Dispositif selon l'une quelconque des revendications précédentes, dans lequel ladite seconde partie de canal sensiblement rectiligne (68) comporte des moyens de restriction (725).

## Patentansprüche

1. Pulverinhalationsvorrichtung, aufweisend einen Körper (10), der mit einer Ausgabeöffnung (15), einer Vielzahl von vordosierten Behältern, die jeweils eine auszugebende Pulverdosis enthalten, und Behälteröffnungsmittel (80) zum Öffnen eines Behälters bei jeder Betätigung versehen ist, wobei die Öffnungsmittel Durchstechmittel sind, die eine Nadel aufweisen, die dazu geeignet ist, bei jeder Betätigung einen vordosierten Behälter zu durchstechen, wobei die Vorrichtung einen Verteilungskanal (70) aufweist, der einen Einlass (710), der mit den Durchstechmitteln (80) verbunden ist und die Pulverdosis aus dem geöffneten Behälter erhält, und einen Auslass (720) aufweist, der mit der Ausgabeöffnung (15) verbunden ist, wobei die Durchstechmittel durch Inhalation durch den Benutzer gesteuert werden, so dass ein vordosierter Behälter gleichzeitig durchstochen und geleert wird, wobei das durch die Strömung der Inhalation mitgezogene Pulver den Verteilungskanal (70) durchquert, bevor es durch die Ausgabeöffnung (15) ausgestoßen wird, **dadurch gekennzeichnet, dass** der Verteilungskanal (70) zwei gebogene Kanalteile (71, 72) aufweist, wobei ein erster gebogener Kanalteil (71) durch einen ersten, im Wesentlichen geradlinigen Kanalteil (67) mit dem Einlass (710) verbunden ist, und ein zweiter gebogener Kanalteil (72) durch einen zweiten, im Wesentlichen geradlinigen Kanalteil (68) mit dem Auslass (720) verbunden ist, wobei die beiden gebogenen Kanalteile (71, 72) durch einen dritten, im Wesentlichen geradlinigen Kanalteil (69) miteinander verbunden sind, wobei der Querschnitt des dritten, im Wesentlichen geradlinigen Kanalteils (69) kleiner ist als der Querschnitt des ersten, im Wesentlichen geradlinigen Kanalteils (67).

2. Vorrichtung nach Anspruch 1, wobei der erste und der zweite im Wesentlichen geradlinige Kanalteil (67, 68) im Wesentlichen parallel sind.

3. Vorrichtung nach den Ansprüchen 1 oder 2, wobei der erste gebogene Kanalteil (71) einen Winkel größer oder gleich 90°, vorzugsweise etwa 100°, bildet.

4. Vorrichtung nach einem der vorhergehenden Ansprüche, wobei der zweite gebogene Kanalteil (72) einen Winkel größer oder gleich 90°, vorzugsweise etwa 100°, bildet.

5. Vorrichtung nach einem der vorhergehenden Ansprüche, wobei der Verteilungskanal (70) einen Bodenteil (701) und einen Deckelteil (702) aufweist.

6. Vorrichtung nach Anspruch 5, wobei der Einlass (710) und der erste, im Wesentlichen geradlinige Kanalteil (67) des Verteilungskanals (70) auf dem Bodenteil (701) gebildet sind, der Auslass (720) und der zweite, im Wesentlichen geradlinige Kanalteil (68) auf dem Deckelteil (702) gebildet sind, und die zwei gebogenen Kanalteile (71, 72) und der dritte, im Wesentlichen geradlinige Kanalteil (69) gleichzeitig auf dem Bodenteil (701) und auf dem Deckelteil (702) gebildet sind.

7. Vorrichtung nach einem der vorhergehenden Ansprüche, wobei der erste, im Wesentlichen geradlinige Kanalteil (67) und/oder der zweite, im Wesentlichen geradlinige Kanalteil (68) und/oder der dritte, im Wesentlichen geradlinige Kanalteil (69) einen über ihre jeweilige Länge im Wesentlichen konstanten Querschnitt aufweisen.

8. Vorrichtung nach einem der vorhergehenden Ansprüche, wobei der zweite, im Wesentlichen geradlinige Kanalteil (68) Begrenzungsmittel (725) aufweist.

## Claims

1. A powder inhaler comprising: a body (10) that is provided with a dispenser orifice (15); a plurality of predosed reservoirs each containing a dose of powder for dispensing; and reservoir-opening means (80) for opening a reservoir on each actuation, said opening means being perforator means comprising a needle that is adapted to perforate a predosed reservoir on each actuation; the inhaler further comprising a dispersion channel (70) including an inlet (710) that is connected to said perforator means (80) and that receives the dose of powder from said open reservoir, and an outlet (720) that is connected to said dispenser orifice (15), said perforator means being controlled by the user inhaling, such that a predosed reservoir is simultaneously perforated and emptied, the powder driven by the inhalation flow passing through said dispersion channel (70) before being expelled through the dispenser orifice (15), the inhaler being **characterized in that** said dispersion channel (70) includes two bent channel portions (71, 72), a first bent channel portion (71) that is connected to said inlet (710) via a first substantially-rectilinear channel portion (67), and a second bent channel portion (72) that is connected to said outlet (720) via a second substantially-rectilinear channel portion (68), said two bent channel portions (71, 72) being interconnected via a third substantially-rectilinear channel portion (69), the cross-section of said third substantially-rectilinear channel portion (69) being smaller than the cross-section of said first substantially-rectilinear channel portion (67).

2. An inhaler according to claim 1, wherein said first and second substantially-rectilinear channel portions (67, 68) are substantially parallel.

3. An inhaler according to claim 1 or claim 2, wherein said first bent channel portion (71) forms an angle that is greater than or equal to 90°, preferably about 100°.

4. An inhaler according to any preceding claim, wherein said second bent channel portion (72) forms an angle that is greater than or equal to 90°, preferably about 100°.

5. An inhaler according to any preceding claim, wherein said dispersion channel (70) comprises a base portion (701) and a cover portion (702).

6. An inhaler according to claim 5, wherein the inlet (710) and the first substantially-rectilinear channel portion (67) of the dispersion channel (70) are formed on the base portion (701), the outlet (720) and the second substantially-rectilinear channel portion (68) are formed on the cover portion (702), and said two bent channel portions (71, 72) and said third substantially-rectilinear channel portion (69) are formed both on the base portion (701) and on the cover portion (702).

7. An inhaler according to any preceding claim, wherein said first substantially-rectilinear channel portion (67) and/or said second substantially-rectilinear channel portion (68) and/or said third substantially-rectilinear channel portion (69) have cross-sections that are substantially constant along their respective lengths.

8. An inhaler according to any preceding claim, wherein said second substantially-rectilinear channel portion (68) includes constriction means (725).
